# EUROPEAN PATENT SPECIFICATION

(11) **EP 1 231 933 B1**
(45) Date of publication and mention of the grant of the patent: **26.07.2006**
(21) Application number: 00976435.8
(22) Date of filing: 17.11.2000
(51) Int. Cl.: A61K 38/21, A61K 9/08

(54) **AN AQUEOUS SOLUTION FORMULATION OF ALPHA-INTERFERON**
FORMULIERUNG EINER WÄSSRIGEN INTERFERON-ALPHA LÖSUNG
FORMULATION D'UNE SOLUTION AQUEUSE DE L'INTERFERON ALPHA

(30) Priority: 19.11.1999 KR 9951481
(43) Date of publication of application: 21.08.2002
(73) Proprietor: LG Chem Investment, Ltd., Youngdungpo-Ku Seoul 150-721 (KR)
(72) Inventor: KANG, Young Cheol, Daedeok-gu, Daejeon 306-778 (KR); LEE, Sang-Heon, Chung-ku, Taejon-si 301-080 (KR); HAN, Kyuboem, Yusong-ku, Taejon-si 305-345 (KR)
(74) Representative: HOFFMANN EITLE
(86) International application number: PCT/KR2000/001322
(87) International publication number: WO 2001/035987

(56) References cited:
- EP-A2- 0 736 303
- WO-A1-96/11018

## Description

### FIELD OF THE INVENTION

The present invention relates to a stable aqueous solution formulation of α- interferon (α-IFN), which does not contain human serum albumin.

### DESCRIPTION OF THE PRIOR ART

Most of the proteins easily denature or lose biological activity in aqueous solution. For this reason, many pharmaceutical companies have developed freeze-dried formulations to improve the stability of the protein drug products. However, the freeze-dried formulation of the protein drugs has many shortcomings. Since the freeze-drying process is expensive and requires additional reconstitution step of protein drug before administering the drug to the patient, it is not preferable to liquid formulation in terms of economy or convenience. These points have been also considered in developing formulations of α-IFN.

Interferon (IFN) plays a role in growth, differentiation and function of normal or tumor cells and is one of the protein cytokines that inhibit the multiplication of a variety of viruses. Specifically, IFN controls the activity of natural killer cell (NK), enhances the activity of cytotoxic T lymphocyte and increase the phagocytic activity of macrophage. Thereby, IFN ultimately mediates immune reaction of the cells infected by virus, etc. IFN can be categorized into α, β and γ, depending on kinds of the secreting cells or inducing materials. Among them, α and β forms are stable even at pH 2, but γ form is unstable under acidic conditions.

Among the three forms, α-interferon has been used as antiviral agent for a long time since it is highly effective in the treatment of viral diseases including hepatitis C. Therefore, many researchers have made continuous efforts to develop formulations which can preserve the activity of α-interferon for a longer period of time.

US patent 4496537 refers to a lyophilization formulation of α-interferon containing alanine (or glycine) and human serum albumin as a stabilizer and a buffer system to maintain the pH in the solution of 6.5 to 8.0. Also, US patent 4847079 refers to an aqueous formulation of α-interferon containing human serum albumin, glycine, thimerosal and a buffer system to maintain the pH at 6.5 to 8.0. Interestingly, both of the above-mentioned patents are characterized in that the formulations contain human serum albumin that is effective in retaining the biological activity of α-interferon. However, when the formulation contains human serum albumin, the possibility that the formulation is potentially contaminated by infectious pathogens such as human immunodeficiency virus (HIV) and hepatitis B virus (HBV) is high. Therefore, recently, it is not recommended to use human serum albumin. Also, it is known that the above albumin can induce immune reactions to some people.

European patent 0736303A2 discloses an aqueous α-interferon formulation containing polysorbate as a stabilizer and benzyl alcohol as an antimicrobial preservative and having a buffer system to maintain pH in the range of 4.5 ~ 5.5. In the above patent, polysorbate, which is safe, is utilized instead of potentially harmful human serum albumin. However, it is known that the antimicrobial activity of benzyl alcohol decreases when used with non-ionic surfactant such as polysorbate 80. In fact, it is discovered that it is not acceptable to use polysorbate 80 with benzyl alcohol. (*Handbook of Pharmaceutical Excipients,* American Pharmaceutical Association, 1986, p18). Therefore, relatively large amount of benzyl alcohol (0.9, 1.0 %) should be used in order to maintain the antimicrobial activity. Such excessive use of benzyl alcohol, however, can cause the aggregation of peptides (Richard L. Remmele et al., *Pharmaceutical Research,* Vol. 15, No. 2, 1998). Also, according to the accelerated test of the formulation of α-interferon at 40 °C observed by the present inventors, the liquid formulation containing 1.0 % benzyl alcohol and 0.02% polysorbate 80 had a much lower activity of α-interferon than the formulation containing phenol (or m-cresol) and 0.02 % polysorbate 80.

WO 96/11018 refers to an aqueous solution formulation containing polysorbate and a chelating agent such as disodium EDTA, and a preservative. However, the above liquid formulation is potentially harmful because it comprises disodium EDTA which is known to be cytotoxic (Paula Saarien-Savolainen et al., *Pharmaceutical Research,* Vol. 15, No. 2, 1998) and it has another problem because it can form chelate complex with calcium ion inside human body. Also, citrate which is mentioned as another chelating agent in the above patent, is known to cause severe pain when administered to the body.

### SUMMARY OF THE INVENTION

To solve the above-mentioned problems, the present inventors have studied to develop an aqueous solution formulation of α-interferon, which retains the biological activity of α-interferon for a long period and is very stable, and which does not contain potentially harmful human serum albumin nor chelating agent. The present inventors have accomplished the present invention by developing an aqueous solution formulation of α-interferon, which can retain the biological activity of α-interferon for a long period and minimize the amount of preservative by means of using polysorbate as a stabilizer and phenol, m-cresol or mixture thereof as an antimicrobial preservative.

It is an object of the present invention to provide a stable aqueous solution formulation of α-interferon, which can retain the biological activity and the physicochemical stability for a long period and can satisfy the acceptance criteria prescribed in the antimicrobial efficacy test of antimicrobial preservation required by European Pharmacopoeia even though it contains only a very small amount of preservatives.

Also, it is another object of the present invention to provide a safe aqueous solution formulation of α-interferon, which retain antimicrobial activities without potentially harmful materials to human body such as human serum albumin or chelating agents.

The present invention provides an aqueous solution formulation of α-interferon consisting of α-interferon; polysorbate 80 as a stabilizer; an osmotic pressure regulating agent: antimicrobial preservatives selected from the group consisting of phenol, m-cresol or mixture thereof; and a buffer system.

In accordance with the present invention, there is provided a stable aqueous solution formulation of α-interferon. Also, there is provided a safer aqueous solution formulation of α-interferon. These and other features, aspects, and advantages of the present invention will become better understood with reference to the following description and appended claims.

### BRIEF DESCRIPTION OF THE DRAWING

Figure 1 is a graph showing the effect of the stabilizer on the biological activity of α-interferon (α-IFN) when the aqueous solution formulation of the present invention is stored for 4 months at 4 °C.
Figure 2a is a graph showing the effect of the antimicrobial preservatives on the stability of the aqueous solution formulation of α-IFN (6 x 10⁶ IU/ml) by measuring the biological activity of α-IFN when the aqueous solution formulation of the present invention is stored for 36 weeks at 4 °C.
Figure 2b is a graph showing the effect of the antimicrobial preservative on the stability of the aqueous solution formulation of α-IFN (6 x 10⁶ IU/ml) by measuring the biological activity of α-IFN when the aqueous solution formulation of the present invention is stored for 16 weeks at 40 °C.
Figure 3a is a graph showing the effect of pH on the stability of the aqueous solution formulation of α-IFN (6 x 10⁶ IU/ml) by measuring the biological activity of α-IFN when the aqueous solution formulation of the present invention is stored for 12 weeks at 40 °C.
Figure 3b is a photograph showing the effect of pH on the dimerization of α-IFN determined by silver-staining the sodium dodecyl sulfate-polyacrylamide gel electrophoresed gel after 12 week-storage at 40 °C.
Figures 4a, 4b and 4c are graphs showing the differences in the biological activities between formulations comprising a mixture of 0.15 % phenol and 0.1 % m-cresol, and formulations comprising 0.3 % phenol alone as an antimicrobial preservative when the aqueous solution formulations of α-IFN (6x10⁶ IU/ml) are stored for 12 weeks at 4 °C, 25 °C and 40 °C, respectively.
Figures 4d, 4e and 4f are graphs showing the differences in the purity determined by reverse phase-high performance liquid chromatography (RP-HPLC) between formulations comprising a mixture of 0.15 % phenol and 0.1 % m-cresol, and formulations comprising 0.3 % phenol alone as an antimicrobial preservative when the aqueous solution formulations of α-IFN (6 x 10⁶ IU/ml) are stored for 12 weeks at 4 °C, 25 °C and 40 °C, respectively.

### DETAILED DESCRIPTION

To achieve the above objects, the present invention provides an aqueous solution formulation of α-interferon consisting of α-interferon; polysorbate 80 as a stabilizer; an osmotic pressure regulating agent, antimicrobial preservatives selected from the group consisting of phenol, m-cresol or mixture thereof ; and a buffer system.

The term " α-Interferon" in the present invention includes all types of α-interferons that are expressed and purified in recombinant bacteria, yeast and animal cells. That is, the term " α-Interferon" as used herein, includes natural and recombinant α-Interferon. Also, α-interferon analogs or variants, where amino acids of natural human α-interferon are partially substituted while more than 50 % of the activity of natural human α-interferon is retained, can be included in the aqueous solution formulation of the present invention. The amount of α-interferon added in the aqueous solution formulation of the present invention is preferably in the range of 1 x 10⁶ IU/ml ~ 1 x 10⁸ IU/ml.

The stabilizer that helps to maintain the stability of α-interferon in the aqueous solution formulation of the present invention is polysorbate 80, and the concentration of polysorbate 80 is preferably in the range of 0.01 ~ 0.05 w/v %.

Also, the aqueous solution formulation of the present invention comprises preservatives including phenol and/or m-cresol to inhibit the growth of microorganisms. Preferable phenol concentration is in the range of 0.1 ~ 0.3 w/v %, preferable m-cresol concentration is in the range of 0.1 ~ 0.2 w/v %, and the appropriate mixture of phenol and m-cresol can also be included in the aqueous solution formulations of the present invention.

The buffer system in the aqueous solution formulation of the present invention includes acetate buffer solution or phosphate buffer solution. More particularly, a buffer system consisting of ammonium acetate and acetic acid, or a buffer system consisting of sodium monohydrogen phosphate (Na₂HPO₄) and sodium dihydrogen phosphate (NaH₂PO₄) is preferable. Also, the concentration of the above buffer system in the aqueous solution formulation of the present invention is preferably in the range of 5 ~ 20 mM. The pH of the aqueous solution formulation of the present invention depends on the above buffer systems. The pH of the aqueous solution formulation of the present invention is in the range of 4.0 ~ 8.0 in most cases and preferably in the range of 4.5 ~ 6.0.

The aqueous solution formulation of the present invention can also include an osmotic pressure regulating agent such as sodium chloride. The amount of the osmotic pressure regulating agent depends on other components in the formulation. Lastly, the aqueous solution formulation of the present invention is prepared under aseptic conditions.

In the present invention, in order to search preferable stabilizers, aqueous solution formulations comprising several excipients useful for the injectable formulations at various concentrations have been prepared, and the effect of the stabilizer on the biological activity of α-Interferon has been studied. The results after storage for 4 months at 4 °C showed that the activities of the aqueous solution formulations comprising human serum albumin, polysorbate 80, polyethylene glycol or gelatin, respectively, as a stabilizer were more than 90 % of the initial filled biological activity before storage while the activity of the aqueous solution formulation comprising only α-Interferon and a buffer system was approximately 80 % of the initial filled biological activity before storage. It is probably because the above stabilizers prevent the adsorption of α-Interferon on the inner surface of a vial. The use of human serum albumin and gelatin, however, is not recommended recently due to the growing concerns on the potential contamination of adventitious viruses like HIV, HBV, etc. Moreover, it may be immunogenic to certain people. Therefore, polysorbate 80 is selected as the most suitable stabilizer in the present invention.

Also, the present inventors have studied the effect of the concentrations of polysorbate 80 and antimicrobial preservatives on the appearance of the aqueous solution formulations. As a result, polysorbate in the range of 0.01 ~ 0.02 % did not affect the clearance of the aqueous solution formulations. However, the aqueous solution formulations became turbid if more than 0.15 % of m-cresol was used, and the degree of the turbidity was proportional to the concentration of m-cresol. When phenol was used as an antimicrobial preservative, phenol in the range of 0.1 ~ 0.3 % did not affect the clearance of the aqueous solution formulations, but the aqueous solution formulations became turbid if more than 0.3 % of phenol was used.

Although the aqueous solution formulation comprising phenol has somewhat greater ability to retain the biological activity than the aqueous solution formulation comprising m-cresol or benzyl alcohol does, the difference is not great. However, in case that the aqueous solution formulation was stored at a high temperature of 40 °C, the biological activity of the aqueous solution formulation comprising benzyl alcohol decreased greatly.

The biological activity as well as dimerization of α-Interferon is influenced by pH. The biological activity of α-Interferon is relatively stable at *ca*. pH 5.8, and dimerization was more frequent at higher pH. Therefore, it is not preferable to have high pH for a long period storage of the aqueous solution formulation of α-Interferon.

Also, the present inventors have investigated antimicrobial preservation effects of the aqueous solution formulations comprising a variety of compositions and concentrations of preservatives according to the protocol to test the efficacy of antimicrobial preparation in European Pharmacopoeia (European Pharmacopoeia 1997, 5.1.3 Efficacy of Antimicrobial Preparation). The concrete protocol to test the efficacy of antimicrobial preparation in European Pharmacopoeia was described in Reference Example 3, and as follows briefly.

Five species of standard strains that are described to use in this test(standard strains in Gram positive and negative bacteria, and fungi Genus) were artificially inoculated at the concentration of 10⁵ to 10⁶ cells per 1 ml of the aqueous solution formulation. At determined time intervals, a portion of the formulations was sampled and the degree of changes in the number of the viable cells were logarithmically transformed. The above logarithmically transformed values (Log reduction values) were compared with the values in the European Pharmacopoeia Table 5.1.3-1 to estimate the level of the antimicrobial activity. The classification between A and B categories described in European Pharmacopoeia can be achieved from the difference in the log reduction values of the microorganisms of interest in course of time. In case of bacteria, A category prescribes that 2 log-reductions within 6 hours and 3 log-reductions in the number of viable micro-organisms against the value obtained for the inoculum within 24 hours should occur, and B category prescribes that 1 log-reduction within 24 hours and 3 log-reductions within 7 days from inoculation should occur. Similarly, in case of fungi, A category prescribes that 2 log-reductions within 7 days should occur, and B category prescribes that 1 log-reduction within 14 days should occur. Also, after the time prescribed above, no recover or increase in the number of viable micro-organisms should be observed. The log-reduction values described above for each category are applied to injectable formulations and eye drops, and the aqueous solution formulation of α-interferon of the present invention falls under this prescription. Therefore, by preparing several aqueous solution formulations of α-interferon comprising different preservatives and estimating the relative antimicrobial activity by performing the antimicrobial efficacy test against 5 species of standard strains, the present inventors have tried to determine the best formulation.

As a result, both the formulation comprising 0.2 % phenol and 0.1 % m-cresol and the formulation comprising 0.15 % phenol and 0.1 % m-cresol satisfied A and B categories in the European Pharmacopoeia against *Aspergillus niger, Candida albicans, Pseudomonas aeruginosa, Staphylococcus aureus* and *Escherichia coli.* However, in case of the formulation comprising 0.15 % phenol and 0.1 % m-cresol, total amounts of preservatives are 0.25% of the formulation. Therefore, the formulation comprising 0.15 % phenol and 0.1 % m-cresol is more preferable formulation in terms of safety, since it has the least amount of the preservatives.

The previously described versions of the present invention have many advantages, including providing a stable aqueous solution formulation of α-interferon which can retain the biological activity of α-interferon for a longer period by preventing the adsorption of α-interferon on the surface of a vial; providing an aqueous solution formulation of α-interferon in which the biological activity as well as the antimicrobial activity of α-interferon can be retained by minimizing amount of the preservatives; providing a safer aqueous solution formulation of α-interferon with a minimum amount of the preservatives because it is known that using a large amount of the preservatives is harmful to human body; providing an aqueous solution formulation of α-interferon which does not contain potentially harmful materials to human body such as human serum albumin or chelating agents; and
providing an aqueous solution formulation of α-interferon which is very stable.

The invention will be further illustrated by the following examples. It will be apparent to those skilled in the art that these examples are given only to illustrate the present invention in more detail, but the invention is not limited to the examples given.

### Reference Example 1: Preparation of purified recombinant α-IFN

Purified α-interferon for the formulation study was prepared by the active substance production process of Intermax-α™ (interferon-α 2a, LG Chemical LTD),

After recombinant *Saccharomyces cerevisiae* containing a human α-interferon gene insertion (*Saccharomyces cerevisiae* pYLBC A/G af α-IFN; Deposit No. KCTC 0051 BP in Korean Collection for Type Cultures of Korea Research Institute of Bioscience and Biotechnology deposited on July 2, 1992) was fermented, alpha-interferon of higher than 95 % reverse phase chromatographic purity was obtained through several purification steps including ion exchange chromatography and gel filtration chromatography. For more detail, please refer to Korean patent application No. 1992-25912, filed December 28, 1992, entitled "Purification process of α-interferon expressed in recombinant yeast", now Korean Patent No. 111251, which is herein incorporated by reference.

### Reference Example 2: Preparation of the aqueous solution formulation of α-interferon and estimation of biological activity thereof

Aqueous solution formulations of α-interferon having the following composition were prepared:
α-Interferon: 1 x 10⁶ IU/ml ~ 100 x 10⁶ IU/ml;
Polysorbate 80: 0.1 ~ 0.5 mg/ml;
Phenol: 1.5 mg/ml;
m-cresol: 1.0 mg/ml; and
a buffer system comprising 10 mM of acetate buffer solution or 10 mM of phosphate buffer solution.

The biological activity of the aqueous solution formulation of α-interferon as prepared above was assayed according to the procedures prescribed in the monograph 1997:1110 'Interferon alfa-2 concentrated solution' of the European Pharmacopoeia. In other words, cell protective effect of the aqueous solution formulation of α-interferon against the cell infection by virus was measured to calculate the International Units (I.U.) by comparing with that of the international standard recombinant human interferon α-2 (IFN α-2) or in-house working standard.

Under the culture condition of 37 °C and 5 % CO₂, MDBK cells (Madin-Derby Bovine Kidney cells: ATCC No. CCL22) were cultured in microtiter plates to form a cell monolayer. And then, more than 3 different concentrations of α-interferon test sample and α-interferon working standard that was calibrated with recombinant human α-interferon international standard from NIH (catalogue number: Gxa 01-901-535, USA) were added to the microtiter plates and cultured. A series of microtiter plates contained cells that were not treated with α-interferon, respectively, and they were used as negative controls. After culturing for 18 ~ 24 hours under the culture condition of 37 °C and 5 % CO₂, the treated α-interferon solution was discarded and cytopathic vesicular stomatitis virus (ATCC No. VR-158) was added into the wells of all the plates except for the controls. After culturing for 24 ~ 48 hours, microtiter plates were stained by crystal violet and dried in the air. After ethylene glycol was added to each well to extract staining dyes, absorbance was measured at 600 nm by using a microplate spectrophotometer.

The absorbance values of standard and test solutions were plotted with respect to the dose of α-interferon to make a linear relationship. Then, the titers of the test solutions were calculated by comparison using parallel line analysis method.

### Reference Example 3: Antimicrobial Efficacy Test of the aqueous solution formulation of α-interferon

Five species of standard strains used for the test were as follow. Two species of fungi strains, *Aspergillus niger* (ATCC 16404) and *Candida albicans* (ATCC 10231) and three species of bacteria strains, *Pseudomonas aeruginosa* (ATCC 9027), *Staphylococcus aureus* (ATCC 6538) and *Escherichia coli* (ATCC 8739) were used.

Agar B (15 g/L Casitone, 5 g/L Soytone, 5 g/L NaCl, 18 g/L agar, pH 7.3 ± 0.2) was used for solid culture of bacteria, and Agar C (10 g/L Peptone, 40 g/L glucose, 15 g/L agar, pH 5.6± 0.2) was used for solid culture of fungi. Bacteria were cultured at 30 ~ 35 °C for 18 ~ 24 hours and fungi were cultured at 20 ~ 25 °C for 24 ~ 48 hours in case of *Candida albicans* and for 2 ~ 7 days in case of *Aspergillus niger.*

In case of *Aspergillus,* black spores formed after culture of about 5 days were collected and used for the test in stead of fungi.

In order to directly inoculate standard strains in a vessel containing the test aqueous solution formulation of α-interferon with the concentration of 10⁵ to 10⁶ cells (spores in case of *Aspergillus)* /ml of standard strains, the cell concentration before inoculation was diluted to 10⁷ to 10⁸ cells (or spores) /ml with dilution buffer, and then the cell suspension diluted was added to the aqueous solution formulation such that quantity of the cell suspension added is 1 (v/v) % of the aqueous solution formulation. As dilution buffer, dilution buffer-A (9 g/L NaCl, 1 g/L Peptone) was used for bacteria and *Candida,* and dilution buffer-B (9 g/L NaCl, 0.5 g/L polysorbate 80) was used for *Aspergillus.*

After the standard strain suspensions were inoculated to the test aqueous solution formulations, each 0.1 ~ 0.5 ml of the test formulations was sampled at 0 hour, 6 hours, 24 hours, 7 days, 14 days and 28 days, respectively, and the solutions sampled were diluted by 1 to 10³ times by using dilution buffers. Then, the diluted samples were smeared onto agar solid medium and cultured at the culture temperatures as described above. Viable cell number per 1 ml of the sample was calculated by counting the colony numbers formed from the solid culture. In the meantime, the test aqueous solution formulations were stored in an incubator at 20 ~ 25 °C immediately after sampling. As the method to estimate viable cell number and the media for the test, those described in European Pharmacopoeia (E.P 1997, 2.6.12) were used.

### Example 1: Search of preferable stabilizers

The experiment below was performed to search preferable stabilizers that retain the biological activity of α-interferon by preventing the adsorption of α-interferon on the surface of a vial.

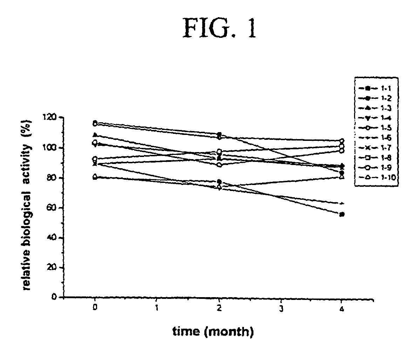

First, aqueous solution formulations comprising several excipients useful for injectable formulations at various concentrations were prepared as described in Table 1, and sodium chloride was added to adjust the tonicity. Each aqueous solution formulation was stored for 4 months at 4 °C to estimate the biological activity of α-interferon in each formulation according to the method described in Reference Example 2. The results are shown in Figure 1 and Table 1 below. Figure 1 is a graph showing the relative biological activity (%) of α-interferon with respect to the filled biological activity of the aqueous solution formulation containing 9 x 10⁶ IU/ml α-interferon.

**Table 1: Real-time storage test at 4 °C**

| Test aqueous formulation | Composition | Control group | | After 2 months | | After 4 months | |
|---|---|---|---|---|---|---|---|
| | | activity | % activity level | Activity | % activity level | Activity | % activity level |
| 1-1 | Buffer solution alone without stabilizer | 7.24 | 80.4% | 7.03 | 78.1% | 5.15 | 57.2% |
| 1-2 | Human serum albumin 0.01 w/v% | 10.5 | 116.7% | 9.85 | 109.4% | 7.64 | 84.9% |
| 1-3 | Human serum albumin 0.5 w/v% | 9.75 | 108.3% | 8.41 | 93.4% | 8.08 | 89.8% |
| 1-4 | Polysorbate 80 0.01 w/v% | 9.21 | 102.3% | 8.65 | 96.1 % | 8.00 | 88.9% |
| 1-5 | Polysorbate 80 0.1 w/v% | 10.4 | 115.6% | 9.63 | 107.0% | -9.55 | 106.0% |
| 1-6 | Polyethyleneglycol4000 0.5 w/v% | 8.07 | 89.7% | 6.60 | 73.3% | 5.79 | 64.3% |
| 1-7 | Polyethyleneglycol6000 0.5 w/v% | 8.06 | 89.6% | 8.39 | 93.2% | 7.95 | 88.3% |
| 1-8 | Gelatin 0.05 w/v% | 8.35 | 92.8% | 8.83 | 98.1% | 9.24 | 102.7% |
| 1-9 | Gelatin 0.2 w/v% | 9.33 | 103.7% | 8.04 | 89.3% | 8.96 | 99.6% |
| 1-10 | Dextran 40T 0.5 w/v% | 7.31 | 81.2% | 6.72 | 74.7% | 7.39 | 82.1% |

| | | | | | | | |
|---|---|---|---|---|---|---|---|
| * Filled biological activity of α-interferon: 9 x 10⁶ IU/ml (unit: x 10⁶ IU/ml) | | | | | | | |

The negative control formulations that comprise only α-interferon and buffer system, retained about 80 % of the initial filled biological activity. On the other hand, the aqueous solution formulation comprising human serum albumin, polysorbate 80, polyethylene glycol or gelatin retained more than 90 % of the initial filled biological activity. It is probably because that the adsorption of α-interferon on the surface of a vial is prevented by the above additives.

Although the above additives contribute to retain the biological activity of α-interferon, the use of human serum albumin and gelatin is restricted for the injectable formulations due to potential contamination problems by the adventitious viruses. Therefore, polysorbate 80 is the most suitable stabilizer.

### Example 2: Effect of the concentrations of polysorbate 80 and antimicrobial preservatives on the appearance of the aqueous solution formulations

As a preliminary experiment for initial selection of the aqueous solution formulation of α-interferon, the effect of the concentrations of polysorbate 80 and antimicrobial preservatives on the appearance of the aqueous solution formulations was investigated. 100 milliliters of the aqueous solution formulations of α-interferon comprising polysorbate 80 and antimicrobial preservatives including phenol, m-cresol or mixture thereof at various concentrations were prepared. Then, the tonicity and pH of each formulation were adjusted by using 0.8 % sodium chloride and 10 mM ammonium acetate buffer, respectively. The pH of each formulation was adjusted to 5.3.

The color and turbidity of the above aqueous solution formulations comprising polysorbate 80 and antimicrobial preservatives at various concentrations were observed by eye. The results are shown in Table 2 below.

**Table 2: Changes in the appearance of the aqueous solution formulations of α-interferon depending on the concentrations of polysorbate 80 and antimicrobial preservatives**

| Test aqueous formulation | Composition | | | Appearance |
|---|---|---|---|---|
| | Polysorbate 80 | Phenol | m-cresol | |
| 2-1 | 0.01 % | - | 0.15% | clear and transparent |
| 2-2 | 0.01 % | - | 0.20% | white and turbid |
| 2-3 | 0.01 % | - | 0.30% | white and turbid |
| 2-4 | 0.01 % | 0.20% | - | clear and transparent |
| 2-5 | 0.01 % | 0.30% | - | clear and transparent |
| 2-6 | 0.01 % | 0.40% | - | white and turbid |
| 2-7 | 0.01 % | 0.50% | - | white and turbid |
| 3-1 | 0.02% | - | 0.15% | clear and transparent |
| 3-2 | 0.02% | - | 0.20% | white and turbid |
| 3-3 | 0.02% | - | 0.30% | white and turbid |
| 3-4 | 0.02% | 0.20% | - | clear and transparent |
| 3-5 | 0.02% | 0.30% | - | clear and transparent |
| 3-6 | 0.02% | 0.40% | - | white and turbid |
| 3-7 | 0.02% | 0.50% | - | white and turbid |
| 3-8 | 0.02% | 0.15% | 0.10% | clear and transparent |
| 3-9 | 0.02% | 0.20% | 0.10% | white and turbid |

When more than 0.15 % of m-cresol was used, the aqueous solution formulations became turbid, and the degree of the turbidity was proportional to the concentration of m-cresol. In case of phenol, the concentration range that did not make the aqueous solution formulations turbid was 0.1 ~ 0.3 %, and the aqueous solution formulations became turbid when more than 0.3 % of phenol was added. The above test results are similar both at the concentration of 0.01 % and at the concentration of 0.02 % of polysorbate 80.

### Example 3: Effect of antimicrobial preservatives on the biological activity of α-interferon

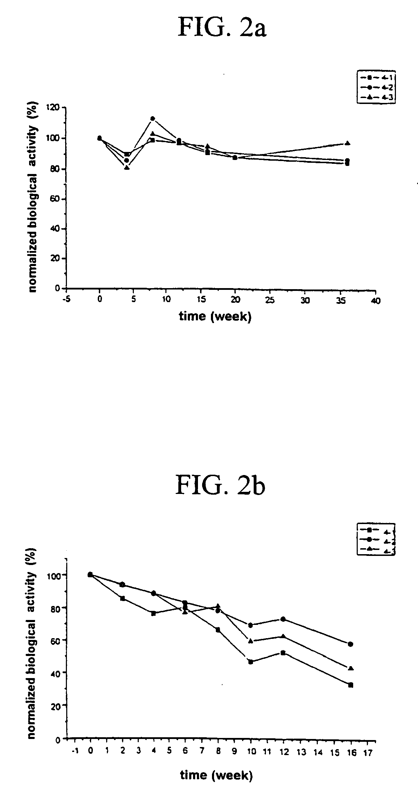

The pH of the aqueous solution formulation was controlled to 4.5 ~ 5.5 by using ammonium acetate buffer, and the tonicity of the aqueous solution formulation was adjusted by adding sodium chloride. The concentration of α-interferon was adjusted to 6 x 10⁶ IU/ml for all aqueous solution formulations. The storage temperature was either at 4 °C or 40 °C. During the storage period, changes in the biological activity of α-interferon were measured by using the method described in Reference Example 2. Table 3 and Figure 2a below shows the result of the biological activity test of the aqueous solution formulation stored at 4 °C, and Table 4 and Figure 2b below shows the result at 40 °C.

**Table 3: Changes in the biological activity of α-interferon during storage of the aqueous solution formulation comprising a variety of antimicrobial preservatives at 4 °C**

| Test aqueous formulation | Preservative (w/v%) | Storage period | | | | | | |
|---|---|---|---|---|---|---|---|---|
| | | 0 | 4 weeks | 8 weeks | 12 weeks | 16 weeks | 20 weeks | 36 weeks |
| 4-1 | Benzyl alcohol (1.0%) | 6.79 | 6.14 | 6.72 | 6.61 | 6.20 | 5.98 | 5.77 |
| | | 100% | 90.4% | 99.0% | 97.4% | 91.3% | 88.1% | 85.0% |
| 4-2 | m-cresol (0.15 %) | 6.51 | 5.58 | 7.35 | 6.46 | 5.96 | - | 5.67 |
| | | 100% | 85.7% | 112.9 % | 99.2% | 91.6% | - | 87.1% |
| 4-3 | Phenol (0.3%) | 6.69 | 5.42 | 6.87 | 6.48 | 6.34 | 5.92 | 6.53 |
| | | 100% | 81.0% | 102.7 | 96.9% | 94.8% | 88.5% | 97.6% |
| Filled biological activity: 6 x 10⁶ IU/ml units: 10⁶ IU/ml | | | | | | | | |

**Table 4: Changes in the biological activity of α-interferon during storage of the aqueous solution formulation comprising a variety of antimicrobial preservatives at 40 °C**

| Test aqueous formulation | Preservative (w/v%) | Storage period | | | | | | | |
|---|---|---|---|---|---|---|---|---|---|
| | | 0 | 2 weeks | 4 weeks | 6 weeks | 8 weeks | 10 weeks | 12 weeks | 16 weeks |
| 4-1 | Benzyl alcohol (1.0 %) | 6.79 | 5.81 | 5.18 | 5.44 | 4.50 | 3.18 | 3.57 | 2.26 |
| | | 100% | 85.6 % | 76.3 % | 80.1 % | 66.3 % | 46.8 % | 52.6 % | 33.3 % |
| 4-2 | m-cresol (0.15 %) | 6.51 | 6.12 | 5.78 | 5.41 | 5.09 | 4.52 | 4.79 | 3.81 |
| | | 100% | 94.0 % | 88.8 % | 83.1 % | 78.2 % | 69.4 % | 73.6 % | 58.5 % |
| 4-3 | Phenol (0.3 %) | 6.69 | 6.27 | 5.94 | 5.14 | 5.41 | 3.97 | 4.19 | 2.9 |
| | | 100% | 93.7 % | 88.8 % | 76.8 % | 80.9 % | 59.3 % | 62.6 % | 43.3 % |
| Filled biological activity: 6 x 10⁶ IU/ml units: 10⁶ IU/ml | | | | | | | | | |

| | | | | | | | | | |
|---|---|---|---|---|---|---|---|---|---|
| * In Tables 3 and 4, the upper value is the absolute biological activity of α-interferon, and the lower value is the relative biological activity (%) of α-interferon with respect to the biological activity of α-interferon at time 0. | | | | | | | | | |

As shown in Table 3 and Figure 2a, although the aqueous solution formulation comprising phenol had slightly higher ability to retain the biological activity than other aqueous solution formulations, the difference was not great. However, in case of storage at 40 °C, the biological activity of the aqueous solution formulation comprising benzyl alcohol decreased greatly in comparison to other aqueous solution formulations (refer to Table 4 and Figure 2b).

### Example 4: Effect of pH on the biological activity and dimerization of α-interferon

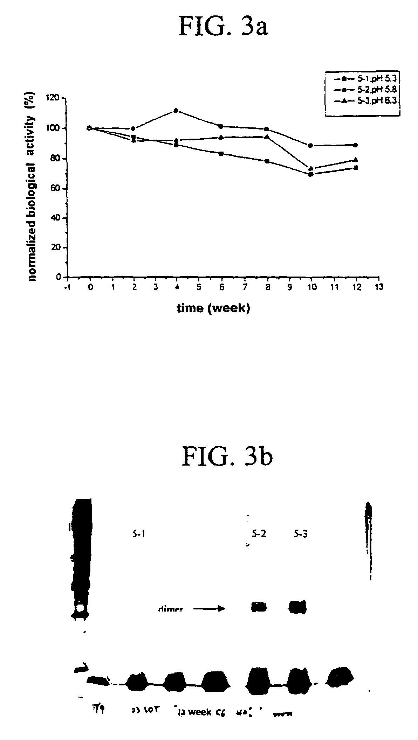

Aqueous solution formulations comprising 6 x 10⁶ IU/ml α-interferon, 0.15 % m-cresol and 10 mM ammonium acetate buffer were prepared. The pH of each aqueous solution formulation was controlled to 5.3, 5.8 and 6.3, respectively. The prepared aqueous solution formulations were stored at 40 °C for 12 weeks, and the biological activity of each aqueous solution formulation was measured at appropriate time intervals. The results were shown in Table 5 and Figure 3a below.

**Table 5: Effect of pH on the biological activity of α-interferon during storage of the aqueous solution formulation at 40 °C**

| Test aqueous formulation | Aqueous formulation pH | Storage period | | | | | | |
|---|---|---|---|---|---|---|---|---|
| | | 0 | 2 weeks | 4 weeks | 6 weeks | 8 weeks | 10 weeks | 12 weeks |
| 5-1 | 5.3 | 6.51 | 6.12 | 5.78 | 5.41 | 5.09 | 4.52 | 4.79 |
| | | 100 % | 94.0% | 88.8% | 83.1% | 78.2% | 69.4% | 73.6 % |
| 5-2 | 5.8 | 6.58 | 6.54 | 7.33 | 6.66 | 6.54 | 5.83 | 5.83 |
| | | 100 % | 99.4% | 111.4 % | 101.2 % | 99.4% | 88.6% | 88.6 % |
| 5-3 | 6.3 | 7.21 | 6.60 | 6.62 | 6.76 | 6.79 | 5.27 | 5.68 |
| | | 100 % | 91.5% | 91.8% | 93.8% | 94.2% | 73.1% | 78.8 % |
| Filled biological activity: 6 x 10⁶ IU/ml units: 10⁶ IU/ml | | | | | | | | |

After 12 weeks, the occurrence and degree of dimerization was measured by 14 % SDS-PAGE (sodium dodecyl sulfate-polyacrylamide gel electrophoresis) (discontinuous buffer system, separating gel; 14 %, 0.375 M Tris-HCI, pH 8.8, stacking gel; 5 %; 0.125M Tris-HCl, pH 6.8) under non-reducing conditions followed by silver-staining.

First, after preparing 14 % separating gel solidified between 2 pieces of glass plates, 5 % stacking gel solution was prepared and poured onto the separating gel, and then a comb was inserted. After the stacking gel is completely solidified, the comb was removed, and each well formed was washed with running buffer (Tris-glycine buffer, pH 8.3). To 100 µl of the above aqueous solution formulation, 50 µl of 3 times concentrated sample buffer (0.186 M Tris, 3 % SDS, 30 v/v% glycerol, 0.009 % bromophenol blue, pH 6.8) was added and mixed well. Then, the mixture was boiled in a boiling water-bath for 2 minutes and cooled in a cold bath. 50 µl of this sample and 10 µl of molecular weight marker (Bio-Rad, low range MW marker) were loaded into different wells. After loading the sample and molecular weight marker, the gel was placed in the electrophoresis system (Hoeffer Scientific Instruments, SE 600) and then running buffer was poured into the system. By applying 10 mA of current per a sheet of gel by connecting the system to the power supply device (Hoeffer Scientific Instruments, PS 2500), electrophoresis was carried out until the bromophenol blue band reached the border between separating gel and stacking gel. When bromophenol blue band reached the border, the current was raised to 20 mA, and then electrophoresis was carried out until the bromophenol blue band reached to the lower end of the glass plate. After the electrophoresis was completed, the power was shut off, the gel was taken out from the system, and the gel containing the proteins was fixed in the mixture of methanol : acetic acid : water (50:10:40) at room temperature for 12 ~ 16 hours. Then, the gel was transferred to a 10 % glutaraldehyde solution for 30 minutes with stirring and washed 3 times with deionized water for each 20 min. After preparing the silver nitrate solution (0.8 % silver nitrate, 0.08 % NaOH, and 4 % ammonia water), the gel was transferred to this solution and agitated in the dark place for 5 minutes. Then, the gel was washed 3 times with deionized water for each 1 min. Developing solution (0.005 % citric acid, 0.14 % formaldehyde, and 0.005 % methanol) prepared immediately before use was added to the gel, and the gel was shaken gently to develop the silver-stained protein bands. The results were shown in Table 6 and Figure 3b below.

**Table 6: Effect of pH on the dimerization of α-interferon**

| Test aqueous formulation | Aqueous formulation pH | **Storage period** | | | | | | |
|---|---|---|---|---|---|---|---|---|
| | | 0 | 2 weeks | 4 weeks | 6 weeks | 8 weeks | 10 weeks | 12 weeks |
| 5-1 | 5.3 | - | - | - | - | - | - | - |
| 5-2 | 5.8 | - | + | + | + | + | ++ | ++ |
| 5-3 | 6.3 | - | + | + | + | ++ | ++ | ++ |
| * - : dimer was not found | | | | | | | | |
| + : lower than 1 % of dimer was found | | | | | | | | |
| ++ : 1 % or more than 1 % of dimer was found | | | | | | | | |

As shown in Table 5 and Figure 3a, the biological activity of α-interferon was relatively stable at ca. pH 5.8. In the meantime, dimerization of α-interferon was more frequent at higher pH (refer to Table 6 and Figure 3b). Consequently, high pH is not preferable for long period-storage of the aqueous solution formulation of α-interferon.

### Example 5: Effect of two preservatives on the antimicrobial activity

Antimicrobial efficacy tests were carried out for the aqueous solution formulations of α-interferon comprising phenol and/or m-cresol as preservatives according to the procedures described in European Pharmacopoeia (E.P 1997, 5.1.3). First, each aqueous solution formulation comprising 6 x 10⁶ IU/ml α-interferon, 0.02 % polysorbate 80 and 10 mM ammonium acetate buffer was prepared. To the formulation, the preservatives at various concentrations as shown in Table 7 below were added, and sodium chloride was added to adjust the tonicity of each formulation. The results of the antimicrobial efficacy test of the aqueous solution formulations are shown in Table 7 below. Viable cell number per 1 ml of aqueous solution formulation was measured by using the procedures as described in Reference Example 3. The viable cell numbers measured at each sampling time were logarithmically transformed, and Log reduction at each time interval was calculated. The Log reductions obtained were compared with the values of criteria A or B prescribed in the European Pharmacopoeia to estimate whether they satisfy the criteria A or B.

**Table 7: Antimicrobial efficacy test of the aqueous solution formulations comprising preservatives at various concentrations**

| Test aqueous formulation | Preservative (w/v) % | Results | | | | |
|---|---|---|---|---|---|---|
| | | *A. niger* | *C. albicans* | *P. aeruginosa* | *S*. *aureus* | *E. coli* |
| 6-1 | 0.15% m-cresol | A & B | A & B | A & B | B | A & B |
| 6-2 | 0.10% m-cresol | A & B | A & B | B | ≠A | ≠A |
| | | | | | ≠B | ≠B |
| 6-3 | 0.075% m-cresol | A & B | A & B | A & B | ≠A | - |
| | | | | | ≠B | |
| 6-4 | 0.30% phenol | A & B | A & B | A & B | A & B | B |
| 6-5 | 0.20% phenol | A & B | A & B | A & B | B | ≠A |
| | | | | | | ≠B |
| 6-6 | 0.15% phenol | A & B | A & B | B | B | - |
| 6-7 | 0.20% phenol + 0.10% m-cresol | A & B | A & B | A & B | A & B | A & B |
| 6-8 | 0.15% phenol + 0.10 % m-cresol | A & B | A & B | A & B | A & B | A & B |
| 6-9 | 0.15 % phenol + 0.075 % m-cresol | - | - | - | B | - |
| 6-10 | 0.20 % phenol + 0.05 % m-cresol | - | - | - | B | - |
| 6-11 | 0.10 % phenol + 0.10 % m-cresol | - | - | - | B | - |

| | | | | | | |
|---|---|---|---|---|---|---|
| * A: it means that the aqueous solution formulation satisfies the criteria A in the European Pharmacopoeia. | | | | | | |
| B: it means that the aqueous solution formulation satisfies the criteria B in the European Pharmacopoeia. | | | | | | |
| - : it means that the test was not performed. | | | | | | |

As can be seen from the results in Table 7, the aqueous solution formulation comprising both 0.15 % phenol and 0.1 % m-cresol satisfied criteria A and B of antimicrobial efficacy test in European Pharmacopoeia even though it contained the least amount of the preservatives (total amount of preservatives: 0.25 %).

### Example 6: Comparison test of the stability between the aqueous solution formulation comprising a mixture of 0.15 % phenol and 0.1 % m-cresol and the aqueous solution formulation comprising 0.3 % phenol alone

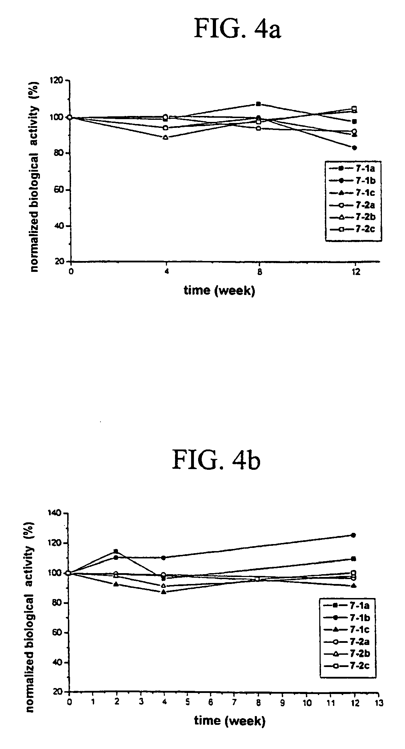

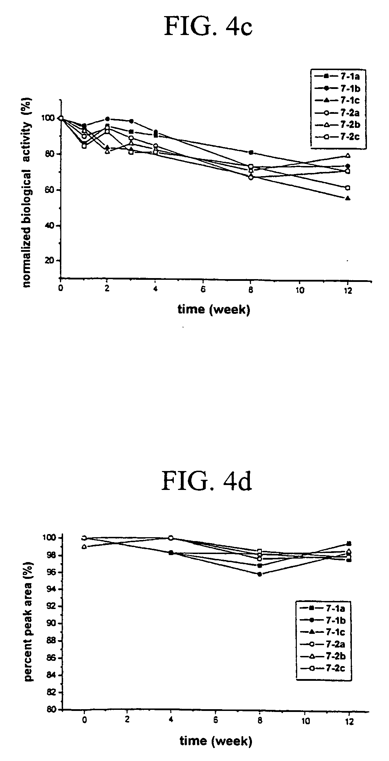

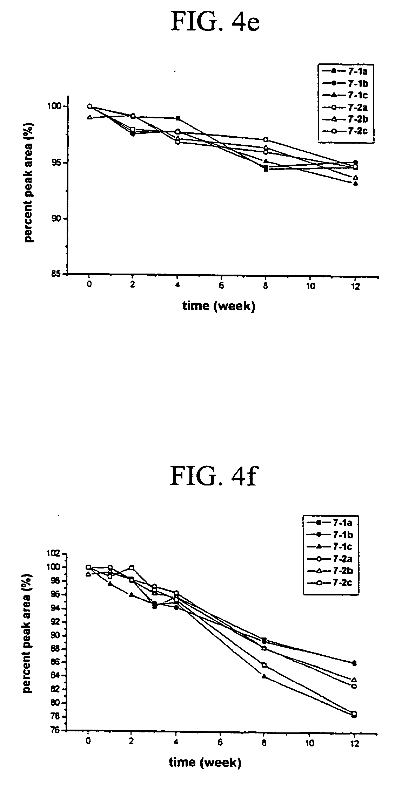

An aqueous solution formulation comprising 6 x 10⁶ IU/ml α-interferon, 0.02 % polysorbate 80, 0.3% phenol, and 10 mM ammonium acetate buffer (Test aqueous solution formulation # 7-1) and the same formulation as described above except that a mixture of 0.15% phenol and 0.1% m-cresol was used instead of 0.3% phenol (Test aqueous solution formulation # 7-2), were prepared. The pH of each aqueous solution formulation was adjusted to 5.3, and sodium chloride was added to the aqueous solution formulation to adjust the tonicity. The two kinds of formulations were divided into 3 groups and the three groups at 4 °C, 25 °C and 40°C, respectively, were stored for 12 weeks. Each group has 3 lots. At regular time intervals, the changes in the biological activity of α-interferon were measured. Purity was determined by using reverse phase-high performance liquid chromatography (RP-HPLC, Waters Alliance System, UV detector, Jupiter C18 column). The results are shown in Tables 8 ~ 13 and Figures 4a ~ 4f below.

### (6-1) Estimation of the changes in the biological activity of α-interferon

**Table 8: Biological activity of α-interferon during storage at 4 °C**

| Test aqueous formulation | Preservative (w/v)% | Storage period | | | |
|---|---|---|---|---|---|
| | | 0 | 4 weeks | 8 weeks | 12 weeks |
| 7-1a | Phenol 0.3 % | 6.11 | 6.05 | 6.58 | 5.98 |
| | | 100.0% | 99.0% | 107.7% | 97.9% |
| 7-1b | Phenol 0.3 % | 6.53 | 6.57 | 6.54 | 5.43 |
| | | 100.0% | 100.6% | 100.2% | 83.2% |
| 7-1c | Phenol 0.3 % | 7.94 | 7.48 | 7.94 | 7.18 |
| | | 100.0% | 94.2% | 100.0% | 90.4% |
| 7-2a | Phenol 0.15 % + m-cresol 0.1 % | 6.68 | 6.69 | 6.29 | 6.18 |
| | | 100.0% | 100.1 % | 94.2% | 92.5% |
| 7-2b | Phenol 0.15 % + m-cresol 0.1 % | 7.77 | 6.89 | 7.67 | 8.06 |
| | | 100.0% | 88.7% | 98.7% | 103.7% |
| 7-2c | Phenol 0.15 % + m-cresol 0.1 % | 6.39 | 6.02 | 6.24 | 6.72 |
| | | 100.0% | 94.2% | 97.7% | 105.2% |

**Table 9: Biological activity of α-interferon during storage at 25 °C**

| Test aqueous formulation | Preservative (w/v)% | Storage period | | | | |
|---|---|---|---|---|---|---|
| | | 0 | 2 weeks | 4 weeks | 8 weeks | 12 weeks |
| 7-1a | Phenol 0.3 % | 6.11 | 6.94 | 5.85 | - | 6.72 |
| | | 100.0% | 113.6% | 95.7% | - | 110.0% |
| 7-1b | Phenol 0.3 % | 6.53 | 7.18 | 7.20 | - | 8.26 |
| | | 100.0% | 110.0% | 110.3% | - | 126.5% |
| 7-1c | Phenol 0.3 % | 7.94 | 7.33 | 6.87 | 7.66 | 7.34 |
| | | 100.0% | 92.3% | 86.5% | 96.5% | 92.4% |
| 7-2a | Phenol 0.15 % + m-cresol 0.1 % | 6.68 | 6.63 | 6.60 | - | 6.49 |
| | | 100.0% | 99.3% | 98.8% | - | 97.2% |
| 7-2b | Phenol 0.15 % + m-cresol 0.1 % | 7.77 | 7.59 | 7.07 | - | 7.67 |
| | | 100.0% | 97.7% | 91.0% | - | 98.7% |
| 7-2c | Phenol 0.15 % + m-cresol 0.1 % | 6.39 | - | - | 6.14 | 6.45 |
| | | 100.0% | - | - | 96.1% | 100.9% |

**Table 10: Biological activity of α-interferon during storage at 40 °C**

| Test aqueous formulation | Preservative (w/v) % | Storage period | | | | | | |
|---|---|---|---|---|---|---|---|---|
| | | 0 | 1 week | 2 weeks | 3 weeks | 4 weeks | 8 weeks | 12 weeks |
| 7-1a | Phenol 0.3 % | 6.11 | 5.28 | 5.89 | 5.68 | 5.56 | 5.02 | 4.39 |
| | | 100.0 % | 86.4 % | 96.4 % | 93.0 % | 91.0 % | 82.2 % | 71.8 % |
| 7-1b | Phenol 0.3 % | 6.53 | 6.24 | 6.56 | 6.47 | 6.06 | 4.84 | 4.88 |
| | | 100.0 % | 95.6 % | 100.5 % | 99.1 % | 92.8 % | 74.1 % | 74.7 % |
| 7-1c | Phenol 0.3 % | 7.94 | 7.55 | 6.69 | 6.61 | - | 5.49 | 4.49 |
| | | 100.0 % | 95.1 % | 84.3 % | 83.2 % | - | 69.1 % | 56.5 % |
| 7-2a | Phenol 0.15 % + m-cresol 0.1% | 6.68 | 6.02 | 6.34 | 6.71 | 6.46 | 5.59 | 6.27 |
| | | 100.0 % | 90.1 % | 94.9 % | 89.5 % | 85.3 % | 68.1 % | 72.2 % |
| 7-2b | Phenol 0.15 % + m-cresol 0.1 % | 7.77 | 7.23 | 6.34 | 6.71 | 6.46 | 5.59 | 6.27 |
| | | 100.0 % | 93.1 % | 81.6 % | 86.4 % | 83.1 % | 71.9 % | 80.7 % |
| 7-2c | Phenol 0.15% + m-cresol 0.1 % | 6.39 | 5.40 | 5.94 | 5.20 | 5.22 | 4.75 | 4.02 |
| | | 100.0 % | 84.5 % | 93.0 % | 81.4 % | 81.7 % | 74.3 % | 62.9 % |

| | | | | | | | | |
|---|---|---|---|---|---|---|---|---|
| ● In Tables 8 ~ 10, the upper value is the absolute biological activity of α-interferon, and the lower value is the relative biological activity (%) of α-interferon with respect to the biological activity of α-interferon at time 0. The unit of each value in the Tables is 10⁶ IU/ml. | | | | | | | | |

### (6-2) Purity of α-interferon

**Table 11: Purity of the aqueous solution formulations during storage at 4 °C determined by RP-HPLC**

| Test aqueous formulation | Preservative (w/v)% | Storage period | | | |
|---|---|---|---|---|---|
| | | 0 | 4 weeks | 8 weeks | 12 weeks |
| 7-1a | Phenol 0.3 % | 100.0% | 98.3% | 96.9% | 99.6% |
| 7-1b | Phenol 0.3 % | 100.0% | 98.3% | 95.9% | 98.5% |
| 7-1c | Phenol 0.3 % | 100.0% | 98.3% | 98.3% | 97.7% |
| 7-2a | Phenol 0.15 % + m-cresol 0.1 % | 100.0% | 100.0% | 97.7% | 98.1 % |
| 7-2b | Phenol 0.15 % + m-cresol 0.1 % | 99.0% | 100.0% | 98.2% | 98.7% |
| 7-2c | Phenol 0.15 % + m-cresol 0.1 % | 100.0% | 100.0% | 98.6% | 98.0% |

**Table 12: Purity of the aqueous solution formulations during storage at 25 °C determined by RP-HPLC**

| Test aqueous formulation | Preservative (w/v) % | Storage period | | | | |
|---|---|---|---|---|---|---|
| | | 0 | 2 weeks | 4 weeks | 8 weeks | 12 weeks |
| 7-1a | Phenol 0.3 % | 100.0% | 99.1 % | 99.0% | 94.6% | 94.8% |
| 7-1b | Phenol 0.3 % | 100.0% | 97.6% | 97.9% | 94.8% | 95.3% |
| 7-1c | Phenol 0.3 % | 100.0% | 97.8% | 97.8% | 95.3% | 93.4% |
| 7-2a | Phenol 0.15 % + m-cresol 0.1 % | 100.0% | 99.2% | 96.9% | 96.1% | 94.8% |
| 7-2b | Phenol 0.15 % + m-cresol 0.1 % | 99.0% | 99.2% | 97.2% | 96.5% | 93.9% |
| 7-2c | Phenol 0.15 % + m-cresol 0.1 % | 100.0% | 98.0% | 97.8% | 97.2% | 94.9% |

**Table 13: Purity of the aqueous solution formulations during storage at 40 °C determined by RP-HPLC**

| Test aqueous formulation | Preservative (w/v) % | Storage period | | | | | | |
|---|---|---|---|---|---|---|---|---|
| | | 0 | 1 week | 2 weeks | 3 weeks | 4 weeks | 8 weeks | 12 weeks |
| 7-1a | Phenol 0.3 % | 100.0 % | 100. 0% | 98.4% | 94.4 % | 95.8 % | 89.7 % | 86.3 % |
| 7-1b | Phenol 0.3 % | 100.0 % | 100. 0% | 98.1 % | 94.9 % | 94.3 % | 89.4 % | 86.4 % |
| 7-1c | Phenol 0.3 % | 100.0 % | 97.6 % | 96.0% | 94.7 % | 95.0 % | 84.2 % | 78.6 % |
| 7-2a | Phenol 0.15 % + m-cresol 0.1 % | 100.0 % | 100. 0% | 98.3% | 97.3 % | 96.4 % | 88.5 % | 82.9 % |
| 7-2b | Phenol 0.15 % + m-cresol 0.1 % | 99.0 % | 99.4 % | 98.2% | 96.3 % | 95.7 % | 88.4 % | 83.8 % |
| 7-2c | Phenol 0.15 % + m-cresol 0.1 % | 100.0 % | 98.7 % | 100.0 % | 96.8 % | 95.4 % | 85.9 % | 78.9 % |

Figures 4a, 4b and 4c are the graphs showing the changes in the relative biological activity with respect to the biological activity of α-interferon at time 0. As shown in Table 8 ~ 13 and Figures 4a ~ 4f, the aqueous solution formulations comprising a mixture of 0.15 % phenol and 0.1 % m-cresol showed a similar tendency with those comprising 0.3 % phenol alone in terms of activity as well as purity during the entire test period. Also, the two kinds of formulations satisfied the antimicrobial requisites prescribed in the antimicrobial efficacy test in European Pharmacopoeia. However, the aqueous solution formulation comprising a mixture of 0.15 % phenol and 0.1 % m-cresol, is safer to human body than that comprising 0.3 % phenol alone because the former contains smaller amount of the preservatives.

## Claims

1. An aqueous solution formulation of α-interferon consisting of α-interferon; polysorbate 80 as a stabilizer; an osmotic pressure regulating agent; antimicrobial preservatives selected from the group consisting of phenol, m-cresol or a mixture thereof; and a buffer system.

2. The aqueous solution formulation of α-interferon according to Claim 1, wherein the amount of α-interferon added is in the range of 1 x 10⁶ IU/ml - 1 x 10⁸ IU/ml.

3. The aqueous solution formulation of α-interferon according to Claim 1, wherein the concentration of polysorbate 80 is in the range of 0.01 - 0.05 w/v%.

4. The aqueous solution formulation of α-interferon according to Claim 1, wherein the osmotic pressure regulating agent is sodium chloride.

5. The aqueous solution formulation of α-interferon according to Claim 1, wherein the preservative is selected from the group consisting of 0.1 - 0.3 w/v% phenol, 0.1 - 0.2 w/v% m-cresol, or a mixture thereof.

6. The aqueous solution formulation of α-interferon according to Claim 1, wherein the buffer system is a buffer system consisting of ammonium acetate and acetic acid; or a buffer system consisting of sodium monohydrogen phosphate (Na₂HPO₄) and sodium dihydrogen phosphate (NaH₂PO₄).

7. The aqueous solution formulation of α-interferon according to Claim 6, wherein the concentration of the buffer system in the aqueous solution formulation is in the range of 5 - 20 mM.

8. The aqueous solution formulation of α-interferon according to Claim 1, wherein the pH of the formulation is in the range of 4.5 - 6.0.

## Patentansprüche

1. Wäßrige Lösungsformulierung von α-Interferon, bestehend aus α-Interferon; Polysorbat 80 als Stabilisator; einem den osmotischen Druck regulierenden Mittel; antimikrobiellen Konservierungsstoffen ausgewählt aus der Gruppe bestehend aus Phenol, m-Cresol oder einer Mischung davon; und einem Puffersystem.

2. Wäßrige Lösungsformulierung von α-Interferon gemäß Anspruch 1, worin die zugegebene Menge an α-Interferon im Bereich von 1-x 10⁶ IU/ml bis 1 x 10⁸ IU/ml liegt.

3. Wäßrige Lösungsformulierung von α-Interferon gemäß Anspruch 1, worin die Konzentration an Polysorbat 80 im Bereich von 0,01 bis 0,05 G/V% liegt.

4. Wäßrige Lösungsformulierung von α-Interferon gemäß Anspruch 1, worin das den osmotischen Druck regulierende Mittel Natriumchlorid ist.

5. Wäßrige Lösungsformulierung von α-Interferon gemäß Anspruch 1, worin das Konservierungsmittel aus der Gruppe bestehend aus 0,1 bis 0,3 G/V% Phenol, 0,1 bis 0,2 G/V% m-Cresol oder einer Mischung davon ausgewählt ist.

6. Wäßrige Lösungsformulierung von α-Interferon gemäß Anspruch 1, worin das Puffersystem ein Puffersystem bestehend aus Ammoniumacetat und Essigsäure oder ein Puffersystem bestehend aus Natriummonohydrogenphosphat (Na₂HPO₄) und Natriumdihydrogenphosphat (NaH₂PO₄) ist.

7. Wäßrige Lösungsformulierung von α-Interferon gemäß Anspruch 6, worin die Konzentration des Puffersystems in der wäßrigen Lösungsformulierung im Bereich von 5 bis 20 mM liegt.

8. Wäßrige Lösungsformulierung von α-Interferon gemäß Anspruch 1, worin der pH der Formulierung im Bereich von 4,5 bis 6,0 liegt.

## Revendications

1. Formulation de solution aqueuse d'α-interféron consistant en α-interféron ; polysorbate 80 en tant que stabilisant ; un agent régulateur de pression osmotique ; des agents conservateurs antimicrobiens choisis dans le groupe consistant en phénol, m-crésol ou leurs mélanges ; et un système tampon.

2. Formulation de solution aqueuse d'α-interféron selon la revendication 1, dans laquelle la quantité d'α-interférons ajoutés se situe dans la plage de 1 x 10⁶ IU/ml - 1 x 10⁸ IU/ml.

3. Formulation de solution aqueuse d'α-interféron selon la revendication 1, dans laquelle la concentration de polysorbate 80 se situe dans la plage de 0,01 - 0,05 % en poids/volume.

4. Formulation de solution aqueuse d'α-interféron selon la revendication 1, dans laquelle l'agent régulant la pression osmotique est le chlorure de sodium.

5. Formulation de solution aqueuse d'α-interféron selon la revendication 1, dans laquelle l'agent conservateur est choisi dans le groupe consistant en 0,1 - 0,3 % en poids/volume de phénol, 0,1 - 0,2 % en poids/volume de m-crésol ou leurs mélanges.

6. Formulation de solution aqueuse d'α-interféron selon la revendication 1, dans laquelle le système tampon est un système tampon consistant en acétate d'ammonium et en acide acétique ; ou un système tampon consistant en phosphate de sodium monohydrogéné (Na₂HPO₄) et de phosphate de sodium dihydrogéné (NaH₂PO₄).

7. Formulation de solution aqueuse d'α-interféron selon la revendication 6, dans laquelle la concentration du système tampon dans la formulation de solution aqueuse se situe dans la plage de 5 - 20 mM.

8. Formulation de solution aqueuse d'α-interféron selon la revendication 1, dans laquelle le pH de la formulation se situe dans la plage de 4,5 - 6,0.
